# EUROPÄISCHE PATENTANMELDUNG

(11) **EP 0 698 378 A1**
(43) Veröffentlichungstag der Anmeldung: **28.02.1996**
(21) Anmeldenummer: 95112181.3
(22) Anmeldetag: 03.08.1995
(51) Int. Cl.: A61B 19/08

(54) **Abdeck- und Sammeleinrichtung für medizinische Untersuchungen und Eingriffe an Extremitäten**

(30) Priorität: 24.08.1994 DE 9413607 U
(71) Anmelder: SENGEWALD KLINIKPRODUKTE GmbH, D-83101 Rohrdorf/Thansau (DE)
(72) Erfinder: Weimar, Albert, D-83026 Rosenheim (DE)
(74) Vertreter: Selting, Günther, Dipl.-Ing.

(57) **Zusammenfassung**

Die Erfindung betrifft eine Abdeck- und Sammeleinrichtung (10) für medizinische Untersuchungen und Eingriffe an Extremitäten. Um eine leichte Handhabbarkeit und geringe Herstellungskosten zu ermöglichen, ist der Auffangbeutel (16) aus in Materialebenenerstreckung elastischem Folienmaterial hergestellt. Die Durchtrittsöffnungen (20,22) sind in der Wandung (18) des Auffangbeutels (16) selbst ausgebildet und abdichtend an der Extremität anordenbar.

## Beschreibung

Die Erfindung betrifft eine Abdeck- und Sammeleinrichtung für medizinische Untersuchungen und Eingriffe an Extremitäten.

Bei Untersuchungen und Behandlungen des menschlichen oder tierischen Körpers ist es nicht nur erforderlich, für die Behandlung ein steriles Umfeld zu schaffen, sondern es muß auch dafür gesorgt werden, daß z.B. Spülfluid nicht in den Behandlungsraum gelangt.

Um beiden Zielen gerecht zu werden, werden Abdeck- und Sammeleinrichtungen verwendet, die ein Abdecktuch und einen Auffangbeutel aufweisen. In dem Abdecktuch ist eine Tuchöffnung zum Durchführen einer Extremität vorhanden. Der mit dem Abdecktuch verbundene Auffangbeutel weist am oberen Ende eine Arbeitsöffnung auf und ist mit einer ersten Durchtrittsöffnung und einer zweiten Durchtrittsöffnung für die Extremität versehen. Die Durchtrittsöffnungen sind dabei derart angeordnet, daß ein zu behandelnder Abschnitt der Extremität innerhalb des durch den Auffangbeutel umgrenzten Volumens oder oberhalb der Arbeitsöffnung positionierbar ist.

Bei der bekannten Abdeck- und Sammeleinrichtung besteht der Auffangbeutel aus einem in Materialebenenerstreckung unelastischen Material, weshalb zur Abdichtung der Durchtrittsöffnungen des Auffangbeutels gegenüber der Extremität separate Abdichteinsätze aus flexiblem Gummimaterial nötig sind. Die separaten Abdichteinsätze sind an der Tuchöffnung bzw. an den Durchtrittsöffnungen des Auffangbeutels angeordnet und mit dem Abdecktuch bzw. dem Auffangbeutel verklebt. Die Anbringung der separaten Abdichteinsätze ist sehr arbeitsaufwendig, so daß diese Abdeck- und Sammeleinrichtungen insgesamt hohe Herstellungskosten erfordern.

Da zur Abdichtung an jeder Durchtrittsöffnung und an der Tuchöffnung jeweils Abdichteinsätze erforderlich sind, ist zur Reduzierung der Anzahl separater Abdichteinsätze bei der bekannten Abdeck- und Sammeleinrichtung der Auffangbeutel großflächig mit dem Abdecktuch verbunden, wobei das Abdecktuch und die eine Wand des Auffangbeutels einen gemeinsamen Abdichteinsatz aufweisen. Dadurch wird zwar die Anzahl der Abdichteinsätze reduziert, jedoch gleichzeitig in Kauf genommen, daß der Fertigungsablauf erschwert und die Handhabbarkeit der Abdeck- und Sammeleinrichtung verschlechtert wird.

Der Erfindung liegt die Aufgabe zugrunde, eine leicht handhabbare, in der Herstellung einfache Abdeck- und Sammeleinrichtung zur Verfügung zu stellen.

Die Lösung dieser Aufgabe erfolgt erfindungsgemäß mit den Merkmalen des Patentanspruchs 1.

Bei der erfindungsgemäßen Abdeck- und Sammeleinrichtung ist der Auffangbeutel aus in Materialebenenerstreckung, elastischem Folienmaterial hergestellt, wobei die erste und zweite Durchtrittsöffnung für die Extremität in der Wandung des Auffangbeutels derart ausgebildet sind, daß ihre Ränder an der Extremität abdichtend anlegbar sind. Dadurch, daß die Wandung des Auffangbeutels selbst die Abdichtung des Auffangbeutels gegenüber der Extremität bewirkt, sind separate Abdichteinsätze nicht erforderlich.

Da Abdecktuch und Auffangbeutel nicht durch Klebung in einer engen gegenseitigen Anlagerung gehalten sind, sind relative Lageänderungen zwischen dem Auffangbeutel und dem Abdecktuch beliebig möglich. Durch die Ausbildung des Auffangbeutels aus elastischem Folienmaterial wird somit nicht nur die Herstellung der Abdeck- und Sammeleinrichtung erleichtert, sondern auch die Handhabbarkeit.

Gemäß einer bevorzugten Ausführungsform der Abdeck- und Sammeleinrichtung fluchten die Tuchöffnung und die erste und zweite Durchtrittsöffnung des Auffangbeutels im zusammengelegten Zustand der Abdeck- und Sammeleinrichtung. Durch die fluchtende Ausrichtung der genannten Öffnungen, die aufgrund der gewonnenen weitgehenden räumlichen Unabhängigkeit von Auffangbeutel und Abdecktuch möglich ist, wird die Handhabbarkeit der Abdeck- und Sammeleinrichtung weiter erleichtert, da das Überstülpen der Abdeck- und Sammeleinrichtung über die zu behandelnde Extremität nur noch ein einmaliges Ausrichten der Abdeck- und Sammeleinrichtung erfordert.

Vorzugsweise weist das Abdecktuch eine Dichtmanschette auf, die auf der dem Auffangbeutel abgewandten Seite des Abdecktuchs aufgeklebt ist und an der Tuchöffnung eine ringförmige Abdichtung bildet. Die Abdichtung erleichtert es, am Ort der Behandlung sterile Arbeitsbedingungen zu gewährleisten, ohne daß der Herstellungsaufwand der Abdeck- und Sammeleinrichtung unverhältnismäßig erhöht würde, da sich die Dichtmanschette gemäß der bevorzugten Ausführungsform manschinell applizieren läßt. Ein solches maschinelles Applizieren ist bei der bekannten Ausführungsform mit einer Dichtmanschette, die zwischen dem Abdecktuch und dem Auffangbeutel angeordnet ist, nicht möglich.

Vorzugsweise ist der Auffangbeutel nur an seinem oberen Ende mit dem Abdecktuch verklebt. Durch diese Freiheit des Auffangbeutels ist es möglich, bei der Verwendung der Abdeck- und Sammeleinrichtung den Auffangbeutel nahe an der zu behandelnden Stelle zu positionieren und gleichzeitig das Abdecktuch auf dem Körper des zu behandelnden Patienten zu belassen. Die Gefahr des Zerreißens von Abdecktuch und Auffangbeutel kann dadurch wesentlich verringert werden.

Durch die Herstellung des Auffangbeutels aus einem Folienschlauch kann das Herstellungsverfahren weiter vereinfacht werden.

Vorzugsweise weist der Auffangbeutel einen Bund mit einer Verstärkung aus flexiblem Schaumstoff auf. Die Verstärkung aus Schaumstoff spreizt bei der Verwendung der Abdeck- und Sammeleinrichtung die Arbeitsöffnung des Auffangbeutels auf, so daß bei der Behandlung anfallende Flüssigkeiten von dem Auffangbeutel zuverlässig aufgenommen werden können. Um die Arthroskopie zu ermöglichen, sollte die Arbeitsöffnung eine ausreichende Öffnungsweite aufweisen.

Weitere vorteilhafte Ausgestaltungen und Weiterbildungen der Erfindung ergeben sich aus den Unteransprüchen sowie aus der Beschreibung im Zusammenhang mit der Zeichnung.

Es zeigen:
- Fig. 1: eine erfindungsgemäße Abdeck- und Sammeleinrichtung in perspektivischer Darstellung beim Einsatz während der Behandlung eines Knies,

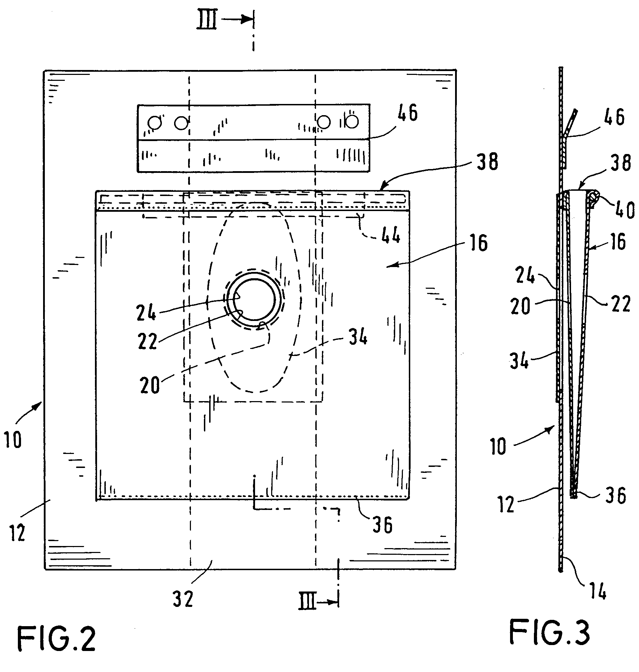

- Fig. 2: die Abdeck- und Sammeleinrichtung gemäß Fig. 1 im zusammengelegten Zustand in einer Ansicht von oben und
- Fig. 3: einen Schnitt durch die Abdeck- und Sammeleinrichtung gemäß der Linie III-III in Fig. 2.

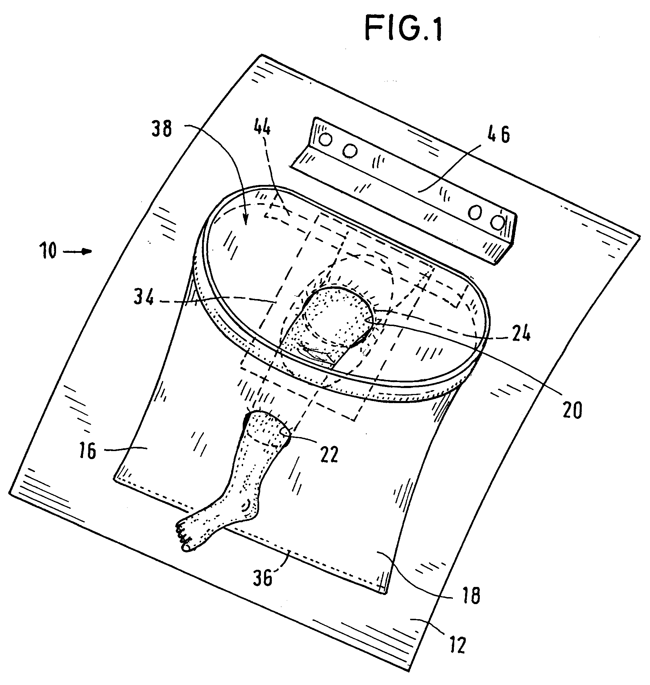

Die in Fig. 1 gezeigte Abdeck- und Sammeleinrichtung 10 besteht aus einem großflächigen, etwa 2 x 3 m großen Abdecktuch 12, an dessen vorderer Seite 14 ein Auffangbeutel 16 angeordnet ist. Der Auffangbeutel 16 weist eine umfangsmäßig geschlossene Beutelwandung 18 auf, in der durch kreisförmiges Ausschneiden eine erste Durchtrittsöffnung 20 und eine dieser gegenüberliegende zweite Durchtrittsöffnung 22 gebildet sind. Der Auffangbeutel 16 ist etwa mittig auf der Oberseite des Abdecktuches 12 angeordnet, wobei sich im zusammengelegten Zustand unterhalb der ersten und zweiten Durchtrittsöffnungen 20,22 mit diesen fluchtend eine Tuchöffnung 24 zum Durchführen einer Extremität befindet.

Das die Tuchöffnung 24 aufweisende Abdecktuch 12 ist ein mehrlagiges, flüssigkeitsundurchlässiges Verbundmaterial. Eine aus gummielastischem Material bestehende Dichtmanschette 34 ist in einem Bereich 32 auf der dem Auffangbeutel 16 abgewandten Seite des Abdecktuchs 12 angeordnet und mit dem Abdecktuch 12 verklebt oder verschweißt.

Der auf der vorderen Seite 14 des Abdecktuchs 12 angeordnete Auffangbeutel 16 besteht aus einem elastischen Folienschlauch und weist an seinem unteren Ende eine verschließende Schweißnaht 36 auf. An seinem oberen Ende ist der Auffangbeutel 16 mit einer Arbeitsöffnung 38 versehen. Die Arbeitsöffnung 38 besitzt einen Bund 40, der durch Umfalten und Abschweißen des Folienschlauches gebildet ist und in dem sich eine Verstärkung aus flexiblem Schaumstoff 42 befindet.

Der Auffangbeutel 16 ist in einem schmalen bandförmigen Bereich 44, der vorzugsweise die Dichtmanschette teilweise überlappt, mit dem Abdecktuch 12 verklebt. Oberhalb des Aufangbeutels 16 ist ein Schlauchhalter 46 aufgeklebt.

Die Abdeck- und Sammeleinrichtung wird im wesentlichen aus zwei Baugruppen hergestellt. Die erste Baugruppe besteht aus dem Abdecktuch 12 und der Dichtmanschette 34, wobei die Dichtmanschette 34 in einem ersten Arbeitsgang gegen das Abdecktuch 12 von dessen Rückseite her geklebt wird. Der Auffangbeutel 16, der die zweite Baugruppe bildet, wird aus einem Folienschlauch hergestellt, indem der Folienschlauch um die Verstärkung 42 herum umgeschlagen und verschweißt wird. Der Folienschlauch wird dann an dem dem Umschlagende abgewandten Ende mit einer Schweißnaht 36 versehen. In den Folienschlauch werden anschließend die erste und zweite Durchtrittsöffnung 20,22 eingeschnitten. Im Anschluß daran werden die erste und die zweite Baugruppe miteinander verklebt. Die einfachen geometrischen Formen von Dichtmanschette 34 und Auffangbeutel 16 erlauben eine maschinelle Fertigung. Abschließend wird die Abdeck- und Sammeleinrichtung 10 verpackt und sterilisiert.

Zum Gebrauch der Abdeck- und Sammeleinrichtung 10 wird diese der Verpackung entnommen. Anschließend werden die Tuchöffnung 24 und die Durchtrittsöffnungen 20,22 über die zu behandelnde Extremität, beispielsweise ein Bein, gestülpt. Die Tuchöffnung 24 und die erste Durchtrittsöffnung 20 werden oberhalb und die zweite Durchtrittsöffnung 21 wird unterhalb des zu untersuchenden Bereichs positioniert. Erst wenn das Abdecktuch 12 mit seiner Tuchöffnung 24 ordnungsgemäß sitzt, wird das Abdecktuch 12 ausgefaltet. Nachdem die Abdeck- und Sammeleinrichtung 10 derart positioniert ist, kann der diagnostische oder operative Eingriff, beispielsweise eine Arthroskopie, durchgeführt werden.

## Patentansprüche

1. Abdeck- und Sammeleinrichtung für medizinische Untersuchungen und Eingriffe an Extremitäten, mit einem eine Tuchöffnung (24) zum Durchführen der Extremität aufweisenden Abdecktuch (12) und einem am oberen Ende eine Arbeitsöffnung (38) aufweisenden Auffangbeutel (16) mit einer ersten Durchtrittsöffnung (20) und mit einer zweiten Durchtrittsöffnung (22) für die Extremität, die derart angeordnet sind, daß ein zu behandelnder Abschnitt der Extremität innerhalb des durch den Auffangbeutel (16) umgrenzten Volumens oder oberhalb der Arbeitsöffnung (38) anordenbar ist,
**dadurch gekennzeichnet**,
daß der Auffangbeutel (16) aus in Materialebenenerstreckung elastischem Folienmaterial hergestellt ist, und daß die zwei Durchtrittsöffnungen (20,22) abdichtend und in der Wandung des Auffangbeutels (16) selbst ausgebildet sind.

2. Abdeck- und Sammeleinrichtung nach Anspruch 1, dadurch gekennzeichnet, daß die Tuchöffnung (24) und die erste und zweite Durchtrittsöffnung (20,22) des Auffangbeutels (16) im zusammengelegten Zustand der Abdeck- und Sammeleinrichtung (10) miteinander fluchten.

3. Abdeck- und Sammeleinrichtung nach Anspruch 1 oder 2, dadurch gekennzeichnet, daß das Abdecktuch (12) eine Dichtmanschette (34) aufweist, die auf der dem Auffangbeutel (16) abgewandten Seite des Abdecktuchs (12) aufgeklebt ist und an der Tuchöffnung (24) eine Abdichtung bildet.

4. Abdeck- und Sammeleinrichtung nach einem der Ansprüche 1 bis 3, dadurch gekennzeichnet, daß der Auffangbeutel (16) nur an seinem oberen Ende mit dem Abdecktuch (12) verklebt ist, so daß sowohl die erste Durchtrittsöffnung (20) als auch die zweite Durchtrittsöffnung (22) im Gebrauchszustand in von der Ebene der Tuchöffnung (24) verschiedenen Ebenen liegen.

5. Abdeck- und Sammeleinrichtung nach einem der Ansprüche 1 bis 4, dadurch gekennzeichnet, daß der Auffangbeutel (16) aus einem Folienschlauch einstückig hergestellt ist.

6. Abdeck- und Sammeleinrichtung nach einem der Ansprüche 1 bis 5, dadurch gekennzeichnet, daß der Auffangbeutel (16) an seiner Arbeitsöffnung (38) einen Bund (40) aufweist, der eine von dem Auffangbeutel (16) allseitig umschlossene Verstärkung (42) aus flexiblem Schaumstoff enthält.

7. Abdeck- und Sammeleinrichtung nach einem der Ansprüche 1 bis 6, dadurch gekennzeichnet, daß die Arbeitsöffnung (38) eine für die Arthroskopie ausreichende Öffnungsweite aufweist.
